# EUROPÄISCHE PATENTANMELDUNG

(11) **EP 0 943 313 A2**
(43) Veröffentlichungstag der Anmeldung: **22.09.1999**
(21) Anmeldenummer: 99100399.7
(22) Anmeldetag: 18.01.1999
(51) Int. Cl.: A61K 7/06

(54) **Haarkosmetische Zubereitungen enthaltend Sonnenblumenwachs und gewünschtenfalls Jojobawachs**

(30) Priorität: 23.01.1998 DE 19802444
(71) Anmelder: Beiersdorf Aktiengesellschaft, 20245 Hamburg (DE)
(72) Erfinder: Argembeaux, Horst, 22525 Hamburg (DE); Koller, Andreas, Dr., 21035 Hamburg (DE); Riedel, Jan-Henric, Dr., 21075 Hamburg (DE)

(57) **Zusammenfassung**

Haarkosmetische Zubereitungen, enthaltend
(a) Sonnenblumenwachs und
(b) gewünschtenfalls Jojobawachs.

## Beschreibung

Die vorliegende Erfindung betrifft haarkosmetische Wirkstoffkombinationen und Zubereitungen, solche Kombinationen enthaltend. Insbesondere betrifft die vorliegende Erfindung haarkosmetische Wirkstoffkombinationen und Zubereitungen zur Pflege des Haars und der Kopfhaut. Die vorliegende Erfindung betrifft insbesondere haarkosmetische Zubereitungen mit einem Gehalt an das Haar sowie die Kopfhaut gegen Oxidationsprozesse schützenden Substanzen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Blechwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Im Laufe der Zeit wird die Oberfläche des Haares durch Umwelteinflüsse und/oder unterschiedlichste chemische und/oder mechanische Behandlungen verändert, insbesondere dergestalt, daß der sogenannte 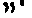 F-layer" abgebaut wird. Das Haar wird dabei immer hydrophiler. Dies läßt sich mit Hilfe der Wilhelmy-Waage und auch der XPS-Technik (ESCA) verifizieren.

Der Stand der Technik ließ es aber an Wirkstoffen und Zubereitungen mangeln, welche das geschädigte Haar in den Urzustand zurückversetzen und so in befriedigender Weise Pflege zukommen lassen können.

Aufgabe war daher, den Nachteilen des Standes der Technik Abhilfe zu schaffen.

Erstaunlicherweise hat sich herausgestellt, daß haarkosmetische Zubereitungen, enthaltend
(a) Sonnenblumenwachs und
(b) gewünschtenfalls Jojobawachs
die Nachteile des Standes der Technik beseitigen.

Die erfindungsgemäßen Zubereitungen pflegen durch Umwelteinflüsse geschädigtes oder strapaziertes Haar bzw. beugen solchen Umwelteinflüssen vor. Zudem verbessern die erfindungsgemäßen Wirkstoffkombinationen und Zubereitungen, diese Wirkstoffkombinationen enthaltend, die Kämmbarkeit des Haares.

Es war ferner überraschend und für den Fachmann nicht vorauszusehen, daß haarkosmetische Zubereitungen, gekennzeichnet durch einen wirksamen Gehalt an Sonnenblumenwachs und gewünschtenfalls Jojobawachs, bzw. die Verwendung von Sonnenblumenwachs und gewünschtenfalls Jojobawachs als gegen schädlichen oxidativen Einfluß auf das menschliche Haar wirksames Prinzip, zumal in haarkosmetischen Zubereitungen den Nachteilen des Standes der Technik abhilft. Unter dem Begriff "oxidativer Einfluß" ist dabei sowohl der Einfluß von oxidierend wirkenden Substanzen zu verstehen als auch die oxidative Wirkung von durch Strahlung, namentlich Licht, und insbesondere UV-Licht, hervorgerufenen Folgeprodukten.

Sonnenblumenwachs fällt als Nebenprodukt bei der Sonnenblumenölraffination an. Üblicherweise hat es folgende Zusammensetzung:

| | | |
|---|---|---|
| ca. | 26 % Wachsester | (hauptsächlich mit 42 - 56 insbesondere 44 - 52 Kohlenstoffatomen) |
| ca. | 58 % Triglyceride | (hauptsächlich von C₁₈-Fettsäuren) |
| ca. | 9 % Diglyceride | (hauptsächlich von C₁₈-Fettsäuren) |
| ca. | 7 % freie Fettsäuren | (hauptsächlich C₁₈-Fettsäuren) |

Jojoba ist eine in der Sonora-Wüste heimische Pflanze aus der Familie der Buxaceen, deren Samen etwa 50 Gew.-% aus Wachs (wegen des niedrigen Festpunktes von ca. 10° C zumeist als 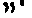 Jojobaöl" bezeichnet) bestehen. Jojobawachs im eigentlichen Sinne ist ein durch Hydrierung entstandes Produkt, welches in den Chemical Abstracts unter den Registraturnummern 61789-91-1 bzw. 66625-78-3 zu erfragen ist. Das durch Hydrierung entstandes Produkt ist jenes, welches im Rahmen der hiermit vorliegenden Offenbarung mit 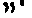 Jojobawachs" bezeichnet wird.

Erfindungsgemäße Zubereitungen enthalten vorteilhaft 0,001 - 25 Gew.-%, bevorzugt 0,005 - 5 Gew.-%, insbesondere bevorzugt 0,01 - 2 Gew.-% an Sonnenblumenwachs, bezogen auf das Gesamtgewicht der Zubereitungen.

Es ist bevorzugt, den erfindungsgemäßen Zubereitungen Jojobawachs einzuverleiben, insbesondere 0,001 - 25 Gew.-%, bevorzugt 0,005 - 5 Gew.-%, insbesondere bevorzugt 0,01 - 2 Gew.-% an Jojobawachs, bezogen auf das Gesamtgewicht der Zubereitungen.

In erstaunlicher und für den Fachmann nicht naheliegender Weise wird durch die Kombination aus Sonnenblumenwachs mit Jojobawachs eine synergistische Wirkung erreicht die wesentlich über die schon beachtliche Wirkung des Sonnenblumenwachses als einzigem der beiden Wachsbestandteile hinausgeht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W) oder vom Typ Öl-in-Wasser-inÖl (O/W/O), ein Gel, eine Hydrodispersion, einen festen Stift, eine Salbe oder auch ein Aerosol darstellen.

Es ist auch möglich und vorteilhaft im Sinne der vorliegenden Erfindung, die erfindungsgemäßen Zubereitungen als wäßrige Systeme bzw. Tensidzubereitungen zur Reinigung und/oder Pflege der Haare auszugestalten.

Erfindungsgemäße Zubereitungen können sowohl der Pflege der Haare und/oder der Kopfhaut dienen als sie auch Verwendung in der dekorativen Kosmetik finden können.

Die erfindungsgemäßen kosmetischen und dermatologischen Zubereitungen können, entsprechend ihrem Verwendungszwecke, kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen und/oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Erfindungsgemäße kosmetische Zubereitungen zur Festigung der Haare, wie z.B. Haarsprays, Haarlacke, Schaumfestiger, Flüssigfestiger, Stylinggele usw., können als Filmbildner grundsätzlich alle für diesen Zweck vorgeschlagenen und bekannten Polymere enthalten, wobei die bevorzugten Einsatskonzentrationen je nach Zubereitung bzw. Polymertyp in der Regel bevorzugt zwischen 0,5 und 20 Gewichtsprozent liegen.

Geeignet sind neben wasserlöslichen oder wasserdispergierbaren Polyurethanen, Polyharnstoffen, Silikonharzen oder Polyestern vor allem die nachfolgend aufgeführten nichtionischen, anionischen, amphoteren und kationischen Polymere, die alleine oder, sofern sie miteinander kompatibel sind, auch in Mischungen mit einem oder mehreren der im folgenden aufgeführten Polymere in alkoholischen, wässrigen bzw. wäßrig-alkoholischen Lösungen eingesetzt werden können.

Nichtionische Polymere, die in den erfindungsgemaßen Zubereitungen alleine oder vorzugsweise auch im Gemisch mit anionischen und/oder amphoteren bzw. zwitterionischen Polymeren verwendet werden, sind insbesondere Vinylpyrrolidon-Homo- oder Copolymerisate. Dazu gehören Polyvinylpyrrolidon, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze.

Weitere erfindungsgemäß geeignete Copolymere sind solche aus Vinylpyrrolidon und Dimethylaminoethyl-methacrylat,Terpolymere aus Vinylcaprolactam, Vinylpyrrolidon und Dimethylaminoethylmethacrylat, Polysiloxane usw.

Geeignete anionische Polymere sind beispielsweise Vinylacetat/Crotonsäure-, Vinylacetat/Acrylat- oder Vinylacetat/Vinylneodecanoat/Crotonsäure-Copolymere, Natriumacrylat/Vinylalkohol-Copolymere, Natriumpolystyrolsulfonat, Ethylacrylat/ N-tert.-Butylacrylamid/ Acrylsäure-Copolymere, Vinylpyrrolidon/Vinylacetat/Itaconsäure-Copolymere, Acrylsäure/Acrylamid-Copolymere oder deren Natriumsalze sowie Homo- und Copolymere von Acrylsäure und Methacrylsäure oder deren Salze. Dazu zählen auch Acrylat/Hydroxyacrylat-, Octylacrylamid/Acrylat- bzw. Methacrylsäurester. Butylacrylat/ N-Vinylpyrrolidon-Copolymere.

Weitere verwendbare anionische Polymere sind Methylvinylether/Maleinsäure-Copolymere, die durch Hydrolyse von Vinylether/Maleinsäureanhydrid-Copolymeren entstehen. Diese Polymeren können auch teilverestert sein (Ethyl, Isopropyl- bzw. Butylester).

Als amphotere Polymere können sowohl alleine als auch im Gemisch mit anionischen und/oder nichtionischen Polymeren Copolymerisate aus N-Octylacrylamid, (Meth)Acrylsäure und tert.-Butylaminoethylmethacrylat vom Typ 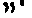 Amphomer", Copolymerisate aus Methacryloylethylbetain und Alkylmethacrylaten vom Typ 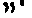 Yukaformer", Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie Mono- bzw. Dialkylaminoalkyl(meth)acrylaten bzw. Mono-bzw. Dialkylaminoalkyl(meth)acrylaten bzw. Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure eingesetzt werden.

Es können selbstverständlich, wie auch bei den anionischen Polymeren, alle in Haarpflegemitteln einsetzbaren amphoteren Polymeren erfindungsgemäß eingesetzt werden.

Zur Verbesserung der Wasserlöslichkeit bzw. der Wasserdispergierbarkeit von anionischen und amphoteren Polymeren werden diese mit geeigneten Basen neutralisiert. Hierzu können Alkali- bzw. Erdalkalibasen, Ammoniak oder verschiedene Amine, wie z.B. Triethanolamin, Triisopropanolamin, Aminomethyl-propanol, Aminomethylpropandiol etc., eingesetzt werden. Die Neutralisation kann je nach Anwendungszweck teilweise oder vollständig erfolgen.

Kationische Polymere, die in den erfindungsgemäßen kosmetischen Zubereitungen eingesetzt werden können, sind u.a. Vinylpyrrolidon/Vinylimidazoliummethochlorid- Copolymere, quaternisierte Vinylpyrrolidon/Dialkylaminoalkylmethacrylat Copolymere, kationische Cellulose-Derivate, wie z.B. Hydroxyethylcellulose/Dimethylalkylammoniumchlorid-Copolymere.

Weiterhin geeignet sind Terpolymere aus Vinylcaprolactam/Vinylpyrrolidon- mit Dimethylaminoethylmethacrylat bzw. Vinylimmidazoliniummethochlorid und Acrylamidocopolymere.

Auch Filmbildner auf natürlicher Basis, z.B. Chitosan und dessen Derivate, können eingesetzt werden, insbesondere im Gemisch mit synthetischen Polymeren.

Die erfindungsgemäßen Zusammensetzungen zur Festigung der Haare können als Haarsprays, Schaumaerosole, Emulsionen, Lösungen oder Gele vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silikone, leicht- und schwerflüchtige verzweigte bzw. unverzweigte Kohlenwasserstoffe, Emulgatoren, Antioxidantien, Wachse Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen, kann das Treibmittel in der Regel entfallen.

Insbesondere können die erfindungsgemäßen kosmetischen und dermatologischen Zubereitungen vorteilhaft Antioxidantien enthalten.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,0001 bis 30 Gew.-%, besonders bevorzugt 0,001 - 10 Gew.-%, insbesondere 0,005 - 1 Gew.-%, bezogen auf das Gesamtgewicht der Zubereitung.

Die wäßrige erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Bei kosmetischen und dermatologischen Zubereitungen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen, Chlorogensäure im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann. Ebenso können die Zubereitungen als Emulsion oder Mikroemulsion vorliegen

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung oder eine Emulsion oder Mikroemulsion dar.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPALaurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats
Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysultain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat sowie Amidopropylbetaine und Alkylbetaine,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Kosmetische Zubereitungen, die kosmetische Reinigungszubereitungen für das Haar bzw. die Kopfhaut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls einen Elektrolyten und Hilfsmittel, wie sie üblicherweise dafür verwendet werden. Die oberflächenaktive Substanz kann in einer Konzentration zwischen 1 und 95 Gew.-% in den Reinigungszubereitungen vorliegen, bezogen auf das Gesamtgewicht der Zubereitungen.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, konditionierende Hilfsmittel, Wirkstoffe und dergleichen.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxy-benzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Es können die für die UVB-Kombination verwendeten Mengen eingesetzt werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1-2

### Conditioner-Shampoo mit Perlglanz

| | 1 | 2 |
|---|---|---|
| Polyquaternium-10 | 0,10 | 0,50 |
| Natriumlaurethsulfat | 11,00 | 11,00 |
| Cocoamidopropylbetain | 2,50 | 2,50 |
| Perlglanzmittel | 2,00 | 2,00 |
| Sonnenblumenwachs | 0,50 | 0,25 |
| Jojobawachs | - | 0,25 |
| Konservierungsmittel, Parfüm, Verdicker, Lösungsvermittler | q.s. | q.s. |
| pH-Einstellung | auf 6,0 | auf 6,0 |
| Wasser, VES | ad 100,00 | ad 100,00 |

### Beispiel 3-4

### klares Conditioner-Shampoo

| | 3 | 4 |
|---|---|---|
| Polyquaternium-10 | 0,10 | 0,50 |
| Natriumlaurethsulfat | 11,00 | 11,00 |
| Cocoamidopropylbetain | 2,50 | 2,50 |
| Sonnenblumenwachs | 0,50 | 0,25 |
| Jojobawachs | - | 0,25 |
| Konservierungsmittel, Parfüm, Verdicker, Lösungsvermittler | q.s. | q.s. |
| pH-Einstellung | auf 6,0 | auf 6,0 |
| Wasser, VES | ad 100,00 | ad 100,00 |

### Beispiel 5-6

### klares Light-Shampoo mit Volumeneffekt

| | 5 | 6 |
|---|---|---|
| Natriumlaurethsulfat | 11,0 | 11,00 |
| Cocoamidopropylbetain | 2,50 | 2,50 |
| Sonnenblumenwachs | 0,50 | 0,25 |
| Jojobawachs | - | 0,25 |
| Konservierungsmittel, Parfüm, Verdicker, Lösungsvermittler | q.s. | q.s. |
| pH-Einstellung | auf 5,5 | auf 5,5 |
| Wasser, VES | ad 100,00 | ad 100,00 |

### Beispiel 7-8

### Haarkur

| | 7 | 8 |
|---|---|---|
| Hydroxypropylmethylcellulose | 0,50 | 0,50 |
| Cetrimoniumbromid | 1,00 | 1,00 |
| Glycerin | 3,00 | 3,00 |
| Cetylstearylalkohol | 2,50 | 2,50 |
| Glycerylstearat | 2,00 | 2,00 |
| Sonnenblumenwachs | 0,50 | 0,25 |
| Jojobawachs | - | 0,25 |
| Konservierungsmittel, Parfüm, Verdicker, Lösungsvermittler | q.s. | q.s. |
| pH-Einstellung | auf 3,5 | auf 3,5 |
| Wasser, VES | ad 100,00 | ad 100,00 |

### Beispiel 9-10

### Haarspülung

| | 9 | 10 |
|---|---|---|
| Behentrimoniumchlorid | 1,00 | 1,00 |
| Glycerin | 3,00 | 3,00 |
| Hydroxyethylcellulose | 0,20 | 0,20 |
| Cetylstearylalkohol | 3,00 | 3,00 |
| Sonnenblumenwachs | 0,50 | 0,25 |
| Jojobawachs | - | 0,25 |
| Konservierungsmittel, Parfüm, Verdicker, Lösungsvermittler | q.s. | q.s. |
| pH-Einstellung | auf 3,0 | auf 3,0 |
| Wasser, VES | ad 100,00 | ad 100,00 |

### Beispiele 11-12

### Schaumfestiger

| | 11 | 12 |
|---|---|---|
| PVP/VA Copolymer | 5,00 | 5,00 |
| Ceteareth-25 | 0,10 | 0,10 |
| Hydroxyethylcetyldimoniumphosphat | 0,10 | 0,10 |
| Sonnenblumenwachs | 0,05 | 0,025 |
| Jojobawachs | - | 0,025 |
| Parfüm, Lösungsvermittler | q.s. | q.s. |
| Ethanol abs. | 10,00 | 10,00 |
| Propan/Butan | 10,00 | 10,00 |
| Wasser, VES | ad 100,0 | ad 100,00 |

### Beispiele 13-14

### Haarspray

| | 13 | 14 |
|---|---|---|
| PVP/VA Copolymer | 6,00 | 6,00 |
| Sonnenblumenwachs | 0,01 | 0,005 |
| Jojobawachs | - | 0,005 |
| Ethanol abs. | 39,00 | 39,00 |
| Wasser, VES | 5,00 | 5,00 |
| Parfüm, Lösungsvermittler | q.s. | q.s. |
| Dimethylether | ad 100,00 | ad 100,00 |

## Patentansprüche

1. Haarkosmetische Zubereitungen, enthaltend
(a) Sonnenblumenwachs und
(b) gewünschtenfalls Jojobawachs.

2. Zubereitungen nach Anspruch 1, enthaltend 0,001 - 25 Gew.-%, bevorzugt 0,005 - 5 Gew.-%, insbesondere bevorzugt 0,01 - 2 Gew.-% an Sonnenblumenwachs, bezogen auf das Gesamtgewicht der Zubereitungen.

3. Zubereitungen nach Anspruch 1, enthaltend 0,001 - 25 Gew.-%, bevorzugt 0,005 - 5 Gew.-%, insbesondere bevorzugt 0,01 - 2 Gew.-% an Jojobawachs, bezogen auf das Gesamtgewicht der Zubereitungen.

4. Verwendung von
(a) Sonnenblumenwachs und
(b) gewünschtenfalls Jojobawachs
zur Pflege des durch Umwelteinflüsse geschädigtes oder strapaziertes Haar bzw. zur Vorbeugung solcher Umwelteinflüsse.

5. Verwendung von
(a) Sonnenblumenwachs und
(b) gewünschtenfalls Jojobawachs
zur Verbesserung der Kämmbarkeit des Haares die Kämmbarkeit des Haares.

6. Verwendung von
(a) Sonnenblumenwachs und
(b) gewünschtenfalls Jojobawachs
als gegen schädlichen oxidativen Einfluß auf das menschliche Haar wirksames Prinzip.
